(19) Europäisches Patentamt
European Patent Office
Office européen des brevets

(11) Veröffentlichungsnummer : **0 429 921 B1**

(12) **EUROPÄISCHE PATENTSCHRIFT**

(45) Veröffentlichungstag der Patentschrift :
**05.01.94 Patentblatt 94/01**

(51) Int. Cl.$^5$ : **C07C 22/04,** C07C 17/14

(21) Anmeldenummer : **90121475.9**

(22) Anmeldetag : **09.11.90**

# (54) Verfahren zur Herstellung von 3-Methyl-benzylchlorid.

(30) Priorität : **22.11.89 DE 3938691**

(43) Veröffentlichungstag der Anmeldung :
**05.06.91 Patentblatt 91/23**

(45) Bekanntmachung des Hinweises auf die Patenterteilung :
**05.01.94 Patentblatt 94/01**

(84) Benannte Vertragsstaaten :
**BE DE FR GB IT**

(56) Entgegenhaltungen :
**JP-A- 5 377 022**

(56) Entgegenhaltungen :
**ULLMANN'S ENCYCLOPEDIA OF INDUSTRIAL CHEMISTRY, 5. Auflage, Band A6, 1986, "Ceramics to Chlorohydrins", Seite 357**
**PATENT ABSTRACTS OF JAPAN, unexamined applications, C Field, Band 6, Nr. 180, 14. September 1982 THE PATENT, OFFICE JAPANESE GOVERNMENT, Seite 113 C 125**

(73) Patentinhaber : **BAYER AG**
**D-51368 Leverkusen (DE)**

(72) Erfinder : **Mais, Franz-Josef, Dr.**
**Gustav-Poensgen-Strasse 23**
**D-4000 Düsseldorf 1 (DE)**
Erfinder : **Fiege, Helmut, Dr.**
**Walter-Flex-Strasse 23**
**D-5090 Leverkusen 1 (DE)**

**EP 0 429 921 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Seitenkettenchlorierung von m-Xylol zur Herstellung von 3-Methylbenzylchlorid, welches durch einen besonders geringen Anteil an kernchlorierten m-Xylolderivaten ausgezeichnet ist.

3-Methyl-benzylchlorid ist ein wertvolles Zwischenprodukt, das bei der Herstellung von Pharmazeutika, Pflanzenschutzmitteln und Riech- und Geschmacksstoffen verwendet wird.

Die Seitenkettenchlorierung von m-Xylol ist aus der allgemeinen chemischen Literatur bekannt (Ullmann's Encyclopedia of Industrial Chemistry, 5. Ed., Vol. A.6 (1986), S. 364 ff.). Die Chlorierung kann entweder durch rein thermische Aktivierung, durch Bestrahlung mit Licht oder UV-Strahlung oder durch eine Kombination aus thermischer Aktivierung und Bestrahlung initiiert werden. Problematisch bei dieser Reaktion ist die Anwesenheit von Eisenspuren, die technisch nur durch außerordentlich hohen Aufwand vermieden werden können. Solche Eisenspuren wirken auch schon in geringsten Mengen als Lewis-Säure-Katalysator, so daß in erheblichem Anteil als störende Nebenreaktion die Kernchlorierung des m-Xylols eintritt. Es ist aus der Literatur bekannt, daß von den 3 isomeren Xylolen, nämlich o-, m- und p-Xylol, gerade das m-Xylol bei weitem die stärkste Neigung zur Kernchlorierung hat (Chemiker-Zeitung 103 (1979), S. 1-7, besonders S. 3, Abschnitt 2.2).

Es ist weiterhin bekannt, daß bei der Seitenkettenchlorierung von Methylaromaten Zusätze zur Vermeidung der Kernchlorierung zugegeben werden können, unter denen auch Pyridin und Pyridin-Derivate genannt sind (Ullmann's Encyclopedia of Industrial Chemistry, 5. Ed., Vol. A.6 (1986), S. 357). Die empfohlenen Konzentrationen liegen bei 0,1 bis 2,0 Gew.-% solcher Zusätze, bezogen auf den zu chlorierenden Methylaromaten. Bei der Seitenkettenchlorierung von kernchlorierten Toluolen wird eine Menge von 0,02-1,0 Gew.-% Pyridin oder substituiertem Pyridin, bezogen auf die Menge des kernchlorierten Toluols, beschrieben (DE-AS 21 39 779).

Bei diesem Stand der Technik und bei der Tatsache, daß m-Xylol außerordentlich leicht zur Kernchlorierung neigt, war zu erwarten, daß zu einer sicheren Vermeidung der unerwünschten Kernchlorierung bei der Monoseitenkettenchlorierung von m-Xylol zumindest die oben beschriebenen Mengen, gegebenenfalls bis in den oberen Bereich von 2 Gew.-% an Pyridin oder Pyridinderivaten angewandt werden müssen.

Aus der JP-A 78-77022 ist bekannt, daß man Xylol in Tetrachlorkohlenstoff an der Seitenkette mit Chlor unter UV-Bestrahlung in der Gegenwart eines Amins chlorieren kann, Konkret ist der Einsatz von Ethanolamin in einer unbekannten Menge offenbart.

Überraschenderweise wurde gefunden, daß gerade eine Absenkung der Menge an Pyridin oder Pyridinderivaten unter die oben genannten Bereiche eine noch weiter erhöhte Selektivität in der gewünschten Richtung ergibt.

Es wurde ein Verfahren zur Herstellung von 3-Methyl-benzylchlorid durch Monoseitenkettenchlorierung von m-Xylol in Gegenwart von Pyridin oder Pyridinderivaten bei erhöhter Temperatur gefunden, das dadurch gekennzeichnet ist, daß man das Pyridin oder Pyridinderivat in einer Menge zwischen 0.00001 und 0,01 Gew.-%, bezogen auf das eingesetzte m-Xylol, anwendet.

Als einzusetzendes Xylol kann ein nicht speziell vom Eisen befreites m-Xylol eingesetzt werden, Man erspart sich daher Vorbehandlungen zur Entfernung von Eisen, wie etwa eine Destillation oder das Ausrühren von Eisen oder Eisenverbindungen mit festem Kaliumhydroxid. Der Wassergehalt des m-Xylols ist für den Reaktionsverlauf ohne besondere Bedeutung. Es kann daher sowohl getrocknetes m-Xylol (beispielsweise durch azeotrope Destillation oder durch Trocknung mit Zeolithen) als auch ein nicht besonders getrocknetes m-Xylol eingesetzt werden. In bevorzugter Weise wird ein m-Xylol mit seinem in der chemischen Technik normalerweise vorkommenden Wassergehalt eingesetzt.

Das erfindungsgemäße Verfahren wird in flüssiger Phase durchgeführt; hierbei kann sowohl in Verdünnung mit einem Lösungsmittel oder ohne ein solches Lösungsmittel gearbeitet werden. Geeignete Lösungsmittel, die durch Chlor unter den Bedingungen der Seitenkettenchlorierung nicht angegriffen werden, sind dem Fachmann bekannt, beispielsweise Perchlorethylen u.a.. In bevorzugter Weise wird das m-Xylol in der unverdünnten flüssigen Form eingesetzt. Als Chloriermittel wird Chlor gasförmig eingeleitet.

Das Pyridin oder Pyridinderivat oder ein Gemisch mehrerer von ihnen wird erfindungsgemäß in einer Menge zwischen 0.00001 und 0,01 Gew.-%, bevorzugt von 0,00005-0,005 Gew.-%, besonders bevorzugt 0,0001-0,001 Gew.-%, bezogen auf das eingesetzte m-Xylol, angewendet. Als Pyridin oder Pyridinderivat wird erfindungsgemäß ein solches der Formel

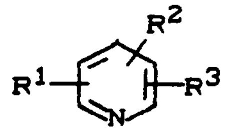

(I)

angewendet, in der

$R^1$, $R^2$ und $R^3$ unabhängig voneinander Wasserstoff, geradkettiges oder verzweigtes $C_1$-$C_8$-Alkyl, geradkettiges oder verzweigtes $C_1$-$C_8$-Alkoxy, Phenyl, Benzyl, Chlor oder Brom bedeuten und $R^1$ zusätzlich Hydroxy sein kann und wobei 2 der Reste $R^1$, $R^2$ und $R^3$, wenn sie benachbart sind, gemeinsam einen kondensierten Benzolring, Trimethylen oder Tetramethylen bedeuten können.

Geradkettiges oder verzweigtes $C_1$-$C_8$-Alkyl ist beispielsweise Methyl, Ethyl, Propyl, Isopropyl, Butyl, Isobutyl, tert.-Butyl, die isomeren Pentyle, Hexyle oder Octyle; bevorzugtes Alkyl hat 1-4 C-Atome; besonders bevorzugt sind Methyl und Ethyl.

Geradkettiges oder verzweigtes $C_1$-$C_8$-Alkoxy ist beispielsweise Methoxy, Ethoxy, Propoxy, Isopropoxy, Butoxy, Isobutoxy, tert.-Butoxy, die isomeren Pentyloxy, Hexyloxy oder Octyloxy; bevorzugt Alkoxy hat 1-4 C-Atome; besonders bevorzugt sind Methoxy und Ethoxy.

Für den Fall, daß 2 der Reste $R^1$, $R^2$ und $R^3$, wenn sie benachbart sind, einen kondensierten Benzolring bedeuten, kommt man in die Reihe der Chinoline oder Isochinoline. Für den Fall, daß 2 der genannten Reste, wenn sie benachbart sind, Trimethylen oder Tetramethylen bedeuten, kommt man in die Reihe der Indano-Pyridine bzw. Cyclohexano-Pyridine.

In bevorzugter Weise wird als Pyridin oder Pyridinderivat ein solches der Formel

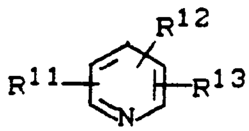

(II)

angewendet, in der

$R^{11}$, $R^{12}$ und $R^{13}$ unabhängig voneinander Wasserstoff, geradkettiges oder verzweigtes $C_1$-$C_4$-Alkyl, geradkettiges oder verzweigtes $C_1$-$C_4$-Alkoxy, Chlor, Brom oder Phenyl bedeuten.

Beispielhaft seien als Verbindungen der Formeln (I) und (II) genannt: Pyridin, 2-Methylpyridin, 2,4-Dimethylpyridin, 2,4,6-Trimethylpyridin, 2-Chlorpyridin, 2-Hydroxypyridin, 4-Phenylpyridin, 2,6-Diphenylpyridin, 2,4,6-Triphenylpyridin, 4-Methoxypyridin, 2-Chlor-5-methylpyridin.

Besonders bevorzugt wird das nicht substituierte Pyridin angewandt.

Als höhere Temperatur für das erfindungsgemäße Verfahren sei eine Temperatur von 50°C bis zum Siedepunkt des Reaktionsgemisches, bevorzugt von 90°C bis zum Siedepunkt des Reaktionsgemisches, besonders bevorzugt von 130°C bis zum Siedepunkt des Reaktionsgemisches, genannt. Das erfindungsgemäße Verfahren kann bei Normaldruck, bei vermindertem oder bei erhöhtem Druck durchgeführt werden. In bevorzugter Weise wird das Verfahren bei Normaldruck oder geringfügig erhöhtem Druck von 1-3 bar durchgeführt.

Das erfindungsgemäße Verfahren kann mit oder ohne Belichtung ausgeführt werden. Wird die Ausführungsform mit Belichtung gewählt, ist eine Belichtung mit künstlichem Licht, welches UV-Strahlung enthält, bevorzugt.

Das erfindungsgemäße Verfahren wird bis zu einem Chloriergrad von 0,8 durchgeführt, wobei der Chloriergrad von 1,0 die Zuführung von 1 Mol Chlor pro 1 Mol m-Xylol bedeutet. Die Fortsetzung der Chlorierung über diesen Chloriergrad von 0,8 hinaus ist grundsätzlich möglich, führt aber wegen des konkurrierenden Auftretens von in den Methylgruppen chlorierten Produkten wieder zur Abnahme von 3-Methyl-benzylchlorid. Daher ist es bevorzugt, einen Chloriergrad von bis zu 0,6, besonders bevorzugt von bis zu 0,3 zu wählen.

Das erfindungsgemäße Verfahren läßt sich sowohl kontinuierlich als auch diskontinuierlich durchführen. Eine beispielsweise Ausführungsform ist die folgende: Man legt in einem Chloriergefäß das m-Xylol vor, gibt die gewünschte Menge an Pyridin oder Pyridinderivaten zu und erhitzt auf die gewünschte Temperatur. Dann wird Chlor, gegebenenfalls unter Belichtung, bis zum gewünschten Chloriergrad eingeleitet. Anschließend wird der Reaktionsansatz durch Ausblasen mit einem Inertgas, beispielsweise mit Stickstoff, von Resten des bei der Chlorierung gebildeten Chlorwasserstoffs befreit und durch Destillation aufgearbeitet, wobei das nicht umgesetzte m-Xylol wieder in die Chlorierung zurückgeführt wird.

Bei dem erfindungsgemäßen Verfahren ist es ausgesprochen überraschend und war bei dem Stand der Technik und der bekannten Tatsache, das m-Xylol außerordentlich leicht zur Kernchlorierung neigt, nicht zu erwarten, daß gerade eine Absenkung der Zusätze an Pyridin und/oder Pyridinderivaten, die die Kernchlorierung verhindern sollen, eine noch weitere Verminderung der Kernchlorierung bewirkt. So ist es nach dem erfindungsgemäßen Verfahren möglich, bei einem technisch vorteilhaften Chloriergrad von 0,3 ohne eine aufwendig trennscharfe Destillation eine gemeinsame Fraktion aus 3-Methyl-benzylchlorid und dem sehr ähnlich siedenden 2-Chlor- und 4-Chlor-m-xylol zu gewinnen, die nur etwa 0,3-0,7 Gew.-% an den unerwünschten kernchlorierten m-Xylolen enthält. Dieses Ergebnis ist technisch sowie ökonomisch und ökologisch außerordentlich vorteilhaft.

Beispiele und Vergleichsbeispiele 1-16

Allgemeine Vorschrift:

In einem schlanken, von außen beheizbaren Chloriergefäß wurden 100 Gew.-Teile m-Xylol vorgelegt und auf die gewünschte Temperatur erhitzt. Nach der Zugabe der gewünschten Menge an Pyridin oder Pyridinderivat wurden 20 Gew.-Teile Chlor (30 Mol-% $Cl_2$, bezogen auf m-Xylol), gegebenenfalls unter Belichtung, gasförmig in einem gleichmäßigen Strom durch eine Bodenfritte im Verlaufe von 2 Stunden eingeleitet.

Die Zusammensetzung des Gemisches wurde durch Gaschromatographie (GC) bestimmt. Das Gemisch wurde nach kurzem Ausblasen mit Stickstoff durch Destillation aufgearbeitet.

Nach dieser allgemeinen Vorschrift wurden die in den folgenden Tabelle angegebenen Beispiele und Vergleichsbeispiele durchgeführt.

**Vergleichsbeispiele 1-4**

a) Reaktionsbedingungen

| Vergleichs-Beispiel Nr. | Gew.-Teile Pyridin | Analytische Gehalte $H_2O$ | Fe | Reaktions-temperatur | Belichtung |
|---|---|---|---|---|---|
| 1 | 0,1 | 58 ppm | <0,05 ppm | 135° C | nein |
| 2 | 0,01 | 164 ppm | <0,05 ppm | 135° C | nein |
| 3 | 0,00001 | 148 ppm | <0,05 ppm | 135° C | nein |
| 4 | ohne Zusatz | 177 ppm | <0,05 ppm | 135° C | nein |

b) Ergebnis der Chlorierung (GC-Prozentgehalte)

| Vergleichs-Beispiel Nr. | Gehalt an 3-Methylbenzyl-chlorid | Gehalt an 2-Chlor- + 4-Chlor-m-xylol | Verhältnis 3-Methylbenzyl-chlorid/2- + 4-Chlor-m-xylol |
|---|---|---|---|
| 1 | 25,10 % | 3,25 % | 7,72 |
| 2 | 33,76 % | 0,63 % | 53,59 |
| 3 | 34,68 % | 0,64 % | 54,19 |
| 4 | 31,07 % | 0,68 % | 45,69 |

**Beispiele 5-6**

a) Reaktionsbedingungen

| Beispiel Nr. | Gew.-Teile Pyridin | Analytische Gehalte $H_2O$ | Analytische Gehalte Fe | Reaktions-temperatur | Belichtung |
|---|---|---|---|---|---|
| 5 | 0,001 | 175 ppm | <0,05 ppm | 135° C | nein |
| 6 | 0,0005 | 162 ppm | <0,05 ppm | 135° C | nein |

b) Ergebnis der Chlorierung (GC-Prozentgehalte)

| Beispiel Nr. | Gehalt an 3-Methylbenzyl-chlorid | Gehalt an 2-Chlor- + 4-Chlor-m-xylol | Verhältnis 3-Methylbenzyl-chlorid/2- + 4-Chlor-m-xylol |
|---|---|---|---|
| 5 | 32,10 % | 0,21 % | 152,86 |
| 6 | 35,91 % | 0,19 % | 189,00 |

<u>Vergleichsbeispiele und Beispiele 7-11</u>

a) Reaktionsbedingungen

| Nr. | Gew.-Teile Pyridin | Analytische Gehalte $H_2O$ | Analytische Gehalte Fe | Reaktions-temperatur | Belichtung* |
|---|---|---|---|---|---|
| Vergleichs-Beispiel 7 | 0,1 | 61 ppm | <0,05 ppm | 133-135° C | ja |
| Beispiel 8 | 0,001 | 61 ppm | <0,05 ppm | 135° C | ja |
| Beispiel 9 | 0,0001 | 167 ppm | <0,05 ppm | 134-135° C | ja |
| Vergleichs-Beispiel 10 | ohne Zusatz | 185 ppm | <0,05 ppm | 135° C | ja |
| Beispiel 11 | 0,001 | 152 ppm | <0,05 ppm | 90° C | ja |

b) Ergebnis der Chlorierung (GC-Prozentgehalte)

| Nr. | Gehalt an 3-Methylbenzyl-chlorid | Gehalt an 2-Chlor- + 4-Chlor-m-xylol | Verhältnis 3-Methylbenzyl-chlorid/2- + 4-Chlor-m-xylol |
|---|---|---|---|
| 7 | 29,47 % | 1,08 % | 27,29 |
| 8 | 32,39 % | 0,23 % | 140,85 |
| 9 | 36,89 % | 0,12 % | 307,42 |
| 10 | 32,51 % | 0,48 % | 67,73 |
| 11 | 37,88 % | 0,16 % | 236,75 |

* Es wurde mit einer 20 Watt-UV-Leuchtstoffröhre belichtet.

**Vergleichsbeispiele und Beispiele 12-14**

a) Reaktionsbedingungen

| Nr. | Gew.-Teile Pyridin | Analytische Gehalte $H_2O$ | Fe | Reaktions-temperatur | Belichtung* |
|---|---|---|---|---|---|
| Vergleichs-Beispiel 12 | ohne Zusatz | 147 ppm | 0,1 ppm | 133-135° C | nein |
| Beispiel 13 | 0,001 | 160 ppm | 0,1 ppm | 133-135° C | nein |
| Beispiel 14 | 0,001 | 158 ppm | 0,1 ppm | 133-135° C | ja* |

b) Ergebnis der Chlorierung (GC-Prozentgehalte)

| Nr. | Gehalt an 3-Methylbenzyl-chlorid | Gehalt an 2-Chlor- + 4-Chlor-m-xylol | Verhältnis 3-Methylbenzyl-chlorid/2- + 4-Chlor-m-xylol |
|---|---|---|---|
| 12 | 28,41 % | 2,77 % | 10,26 |
| 13 | 30,69 % | 0,21 % | 146,14 |
| 14 | 31,98 % | 0,20 % | 159,90 |

* Es wurde mit einer 20 Watt-UV-Leuchtstoffröhre belichtet.

**Beispiele 15-16**

a) Reaktionsbedingungen

| Beispiel Nr. | Zusatz | Gew.-Teile | Analytische Gehalte $H_2O$ | Analytische Gehalte Fe | Reaktions-temperatur | Belichtung |
|---|---|---|---|---|---|---|
| 15 | 2-Methyl-pyridin | 0,001 | 178 ppm | 0,1 ppm | 135° C | nein |
| 16 | 4-Phenyl-pyridin | 0,002 | 149 ppm | 0,1 ppm | 135° C | nein |

b) Ergebnis der Chlorierung (GC-Prozentgehalte)

| Beispiel Nr. | Gehalt an 3-Methylbenzyl-chlorid | Gehalt an 2-Chlor- + 4-Chlor-m-xylol | Verhältnis 3-Methylbenzyl-chlorid/2- + 4-Chlor-m-xylol |
|---|---|---|---|
| 15 | 32,09 % | 0,22 % | 145,86 |
| 16 | 33,35 % | 0,24 % | 139,58 |

## Patentansprüche

1. Verfahren zur Herstellung von 3-Methyl-benzylchlorid durch Monoseitenkettenchlorierung von m-Xylol in Gegenwart von Pyridin oder Pyridinderivaten bei erhöhter Temperatur, dadurch gekennzeichnet, daß man das Pyridin oder Pyridinderivat in einer Menge zwischen 0,00001 und 0,01 Gew.-%, bezogen auf das eingesetzte m-Xylol, anwendet.

2. Verfahren nach Anspruch 1, dadurch gekennzeichnet, daß man das Pyridin oder das Pyridinderivat oder ein Gemisch mehrerer von ihnen in einer Menge von 0,00005-0,005 Gew.-%, bevorzugt von 0,0001-0,001 Gew.-%, bezogen auf das eingesetzte m-Xylol, anwendet.

3. Verfahren nach Anspruch 1, dadurch gekennzeichnet, daß man ein Pyridin oder Pyridinderivat der Formel

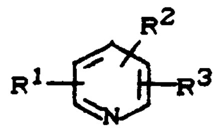

anwendet, in der

$R^1$, $R^2$ und $R^3$ unabhängig voneinander Wasserstoff, geradkettiges oder verzweigtes $C_1$-$C_8$-Alkyl, geradkettiges oder verzweigtes $C_1$-$C_8$-Alkoxy, Phenyl, Benzyl, Chlor oder Brom bedeuten und $R^1$ zusätzlich Hydroxy sein kann und wobei 2 der Reste $R^1$, $R^2$ und $R^3$, wenn sie benachbart sind, gemeinsam einen kondensierten Benzolring, Trimethylen oder Tetramethylen bedeuten können.

4. Verfahren nach Anspruch 3, dadurch gekennzeichnet, daß man ein Pyridin oder Pyridinderivat der Formel

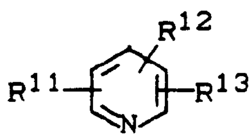

anwendet, in der

$R^{11}$, $R^{12}$ und $R^{13}$ unabhängig voneinander Wasserstoff, geradkettiges oder verzweigtes $C_1$-$C_4$-Alkyl, geradkettiges oder verzweigtes $C_1$-$C_4$-Alkoxy, Chlor, Brom oder Phenyl bedeuten.

5. Verfahren nach Anspruch 4, dadurch gekennzeichnet, daß man Pyridin anwendet.

6. Verfahren nach Anspruch 1, dadurch gekennzeichnet, daß die Chlorierung bei einer Temperatur von 50°C bis zum Siedepunkt des Reaktionsgemisches, bevorzugt bei einer Temperatur von 90°C bis zum Siedepunkt des Reaktionsgemisches, besonders bevorzugt bei einer Temperatur von 130°C bis zum Siedepunkt des Reaktionsgemisches durchgeführt wird.

7. Verfahren nach Anspruch 1, dadurch gekennzeichnet, daß die Chlorierung bei einem Druck von 1-3 bar durchgeführt wird.

8. Verfahren nach Anspruch 1, dadurch gekennzeichnet, daß die Chlorierung unter Belichtung mit UV-Strahlung enthaltendem Licht durchgeführt wird.

9. Verfahren nach Anspruch 1, dadurch gekennzeichnet, daß die Chlorierung bis zu einem Chloriergrad von 0,8, bevorzugt von bis zu 0,6, besonders bevorzugt von bis zu 0,3 durchgeführt wird.

## Claims

1. Process for the preparation of 3-methyl-benzyl chloride by side chain monochlorination of m-xylene in the presence of pyridine or pyridine derivatives at elevated temperature, characterized in that the pyridine or pyridine derivative is used in an amount of between 0.00001 and 0.01 % by weight, based on the m-xylene employed.

2. Process according to Claim 1, characterized in that the pyridine or the pyridine derivative or a mixture of several of these is used in an amount of 0.00005-0.005% by weight, preferably 0.0001-0.001 % by weight, based on the m-xylene employed.

3. Process according to Claim 1, characterized in that a pyridine or pyridine derivative of the formula

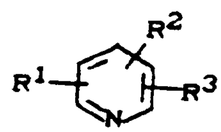

in which
$R^1$, $R^2$ and $R^3$ independently of one another denote hydrogen, straight-chain or branched $C_1$-$C_8$-alkyl, straight-chain or branched $C_1$-$C_8$-alkoxy, phenyl, benzyl, chlorine or bromine and $R^1$ can additionally be hydroxyl, and wherein 2 of the radicals $R^1$, $R^2$ and $R^3$, if these are adjacent, together can denote a fused benzene ring, trimethylene or tetramethylene, is used.

4. Process according to Claim 3, characterized in that a pyridine or pyridine derivative of the formula

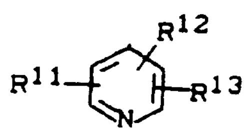

in which
$R^{11}$, $R^{12}$ and $R^{13}$ independently of one another denote hydrogen, straight-chain or branched $C_1$-$C_4$-alkyl, straight-chain or branched $C_1$-$C_4$-alkoxy, chlorine, bromine or phenyl, is used.

5. Process according to Claim 4, characterized in that pyridine is used.

6. Process according to Claim 1, characterized in that the chlorination is carried out at a temperature of 50°C up to the boiling point of the reaction mixture, preferably at a temperature of 90°C up to the boiling point of the reaction mixture, particularly preferably at a temperature of 130°C up to the boiling point of the reaction mixture.

7. Process according to Claim 1, characterized in that the chlorination is carried out under a pressure of 1-3 bar.

8. Process according to Claim 1, characterized in that the chlorination is carried out under exposure to light using light containing UV radiation.

9. Process according to Claim 1, characterized in that the chlorination is carried out up to a degree of chlorination of 0.8, preferably of up to 0.6, particularly preferably of up to 0.3.

## Revendications

1. Procédé pour la préparation de chlorure de 3-méthylbenzyle par monochloration d'une des chaînes latérales du m-xylène en présence de pyridine ou de dérivés de la pyridine à température élevée, caractérisé en ce qu'on utilise la pyridine ou le dérivé de pyridine en une quantité entre 0,00001 et 0,01% en poids, rapportée au m-xylène mis en oeuvre.

2. Procédé selon la revendication 1, caractérisé en ce qu'on utilise la pyridine ou le dérivé de pyridine ou encore un mélange de plusieurs d'entre eux en une quantité de 0,00005-0,005% en poids, de préférence de 0,0001-0,001% en poids, rapportée au m-xylène mis en oeuvre.

3. Procédé selon la revendication 1, caractérisé en ce qu'on utilise une pyridine ou un dérivé de pyridine de formule

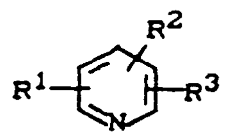

dans laquelle
$R^1$, $R^2$ et $R^3$ représentent, indépendamment l'un de l'autre, un atome d'hydrogène, un groupe alkyle en $C_1$-$C_8$ à chaîne droite ou ramifiée, un groupe alcoxy en $C_1$-$C_8$ à chaîne droite ou ramifiée, un groupe phényle, un groupe benzyle, un atome de chlore ou un atome de brome, et $R^1$ peut représenter, en outre, un groupe hydroxyle, deux des radicaux $R^1$, $R^2$ et $R^3$, lorsqu'ils sont voisins, peuvent représenter ensemble un cycle benzénique condensé, un groupe triméthylène ou un groupe tétraméthylène.

4. Procédé selon la revendication 3, caractérisé en ce qu'on utilise une pyridine ou un dérivé de pyridine de formule

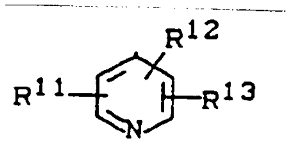

dans laquelle
$R^{11}$, $R^{12}$ et $R^{13}$ représentent,indépendamment l'un de l'autre, un atome d'hydrogène, un groupe alkyle en $C_1$-$C_4$ à chaîne droite ou ramifiée, un groupe alcoxy en $C_1$-$C_4$ à chaîne droite ou ramifiée, un atome de chlore, un atome de brome ou un groupe phényle.

5. Procédé selon la revendication 4, caractérisé en ce qu'on utilise de la pyridine.

6. Procédé selon la revendication 1, caractérisé en ce qu'on effectue la chloration à une température de 50°C jusqu'au point d'ébullition du mélange réactionnel, de préférence à une température de 90°C jusqu'au point d'ébullition du mélange réactionnel, de manière particulièrement préférée à une température de 130°C jusqu'au point d'ébullition du mélange réactionnel.

7. Procédé selon la revendication 1, caractérisé en ce qu'on effectue la chloration sous une pression de 1-3 bar.

8. Procédé selon la revendication 1, caractérisé en ce qu'on effectue la chloration avec exposition à de la lumière contenant des rayons UV.

9. Procédé selon la revendication 1, caractérisé en ce qu'on effectue la chloration jusqu'à ce qu'on obtienne un degré de chloration de 0,8, de préférence jusqu'à 0,6, de manière particulièrement préférée jusqu'à 0,3.